# NEUE EUROPÄISCHE PATENTSCHRIFT

(11) **EP 1 472 447 B2**
(45) Veröffentlichungstag und Bekanntmachung des Hinweises auf die Entscheidung über den Einspruch: **25.04.2018**
(45) Hinweis auf die Patenterteilung: 29.11.2006
(21) Anmeldenummer: 03731614.8
(22) Anmeldetag: 22.01.2003
(51) Int. Cl.: F02C 9/40, F02C 3/22, F23N 5/00, G01N 21/35, G01N 33/00

(54) **VERFAHREN ZUM BETRIEB EINER GASTURBOGRUPPE**
METHOD FOR OPERATING A GAS TURBINE GROUP
PROCEDE D'UTILISATION D'UN GROUPE DE TURBINES A GAZ

(30) Priorität: 25.01.2002 DE 10203102; 10.06.2002 CH 9912002
(43) Veröffentlichungstag der Anmeldung: 03.11.2004
(73) Patentinhaber: Ansaldo Energia IP UK Limited, London W1G 9DQ (GB)
(72) Erfinder: DÖBBELING, Klaus, CH-5210 Windisch (CH); HAFFNER, Ken-Yves, CH-5400 Baden (CH); RÜETSCHI, Rolf, CH-5415 Nussbaumen (CH); ZINN, Hanspeter, CH-5406 Baden-Rütihof (CH)
(74) Vertreter: Bernotti, Andrea
(86) Internationale Anmeldenummer: PCT/CH2003/000045
(87) Internationale Veröffentlichungsnummer: WO 2003/062618

(56) Entgegenhaltungen:
- EP-A- 0 156 200
- EP-A- 0 554 095
- EP-A- 1 022 514
- EP-A- 1 070 955
- WO-A-00/14451
- WO-A-00/52315
- WO-A-02/14661
- WO-A-91/06809
- DE-A- 19 921 981
- DE-A1- 10 022 882
- DE-C1- 19 918 901
- JP-A- 53 038 808
- JP-A- 2000 266 341
- US-A- 4 594 510
- US-A- 6 082 092

## Beschreibung

### Technisches Anwendungsgebiet

Die vorliegende Erfindung betrifft ein Verfahren zum Betrieb einer Gasturbogruppe gemäss dem Oberbegriff des Anspruchs 1. Sie betrifft weiterhin eine Gasturbogruppe zur Durchführung des Verfahrens.

### Stand der Technik

Die Stabilität der Verbrennung in modernen Vormischbrennern von Gasturbinen hängt entscheidend mit von der Zündwilligkeit des verwendeten Brennstoffs, aber auch anderen Brennstoffeigenschaften wie insbesondere dem Heizwert oder dem Wobbe-Index, ab.

Derartige Vormischbrenner sind beispielsweise aus der EP 321 809, der EP 780 629, der WO 93/17279, oder der WO 92/19913 bekanntgeworden. Den Brennerbauarten liegt der gemeinsame Gedanke zugrunde, Brennstoff in einen verdrallten Verbrennungsluftstrom einzubringen und eine möglichst homogene und üblicherweise unterstöchiometrische, magere Brennstoff-Luft-Mischung zu erzeugen. Beim Übergang in den Brennraum platzt die Drallströmung an einem Querschnittsübergang auf, wodurch sich vor der Brennermündung ein Rückströmgebiet ausbildet, welches zur Flammenstabilisierung dient. Dabei darf die Flamme nicht zu nahe an der Brennermündung stabilisiert werden, um eine thermische Überlastung des Brenners zu vermeiden. Wenn die Stabilisierungszone aber zu weit stromab der Brennermündung liegt, kommt es zu Instabilitäten.

Die Lage der Verbrennungszone ist auch entscheidend von der Zündwilligkeit des verwendeten Brennstoffs abhängig. Dieser ändert sich dramatisch, wenn beispielsweise ein Brenngas hohe Anteile an höherwertigen gesättigten Kohlenwasserstoffen, wie Ethan, Butan, Propan, auch als C₂₊-Alkane bezeichnet, aufweist. Da das Brennstoff-Luftgemisch vorgemischt zugeführt wird, besteht die akute Gefahr eines Flammenrückschlages zum Brenner. Ein Bauteilversagen ist dann wahrscheinlich.

Eine ähnliche Problemstellung zeigt sich auch bei beim Betrieb von selbstzündenden Brennkammern des aus EP 669 500 bekannten Typs, beispielsweise in einer Gasturbogruppe mit sequentieller Verbrennung, wie sie aus EP 620 362 bekanntgeworden ist. Auch hier kann ein durch erhöhte Zündwilligkeit des Brenngases hervorgerufener Flammenrückschlag zu einer Grosshavarie führen.

Eine veränderte Brenngaszusammensetzung hat weiterhin auch Auswirkungen auf die Emissionen und auf die Brennkammerpulsationen.

Neben dem Gehalt an C₂₊-Alkanen hat auch der Heizwert oder der Wobbe-Index einen Einfluss auf das Verhalten der Verbrennung. Dieser Einfluss ist in Vormischbrennersystemen in erster Linie auf das veränderte Impulsverhältnis von Brenngas und Luft bei unterschiedlichen Wobbe-Zahlen zurückzuführen. US 6,082,092 gibt an, eine variable Brenngasvorwärmung so zu regeln, dass der Wobbe-Index konstantgehalten wird. Eine Regelung auf einen konstanten Wobbe-Index wäre auch durch die variable Zumischung inerter Komponenten in einem geschlossenen Regelkreis möglich. DE 197 31 209 schlägt vor, bei der Verbrennung von Restgasen stark variierender Zusammensetzung den Wobbe-Index durch eine geregelte Zumischung von Erdgas und Stickstoff konstantzuhalten.

Aus DE 199 21 981 A1 ist bekannt, den Brennstoffheizwert zu bestimmen und nach dieser Massgabe den Brennstoffmassenstrom zu steuern. Aber auch DE 199 21 981 gibt keinen Hinweis darauf, wie einer geänderten Zündwilligkeit des Gases und einer daraus resultierenden Gefahr des Flammenrückschlages zu begegnen sei.

Der Stand der Technik gibt jedoch keinen Hinweis darauf, wie auf eine veränderte Brenngaszusammensetzung hinsichtlich des Gehalts an C₂₊-Alkanen zu reagieren sei. Festzustellen bleibt dabei, dass nachhaltige Beschädigungen durch einen Flammenrückschlag sehr schnell auftreten können. Ein Verfahren, bei dem auf veränderte C₂₊-Alkan-Gehalte im Brenngas einer Gasturbogruppe reagiert werden soll, muss daher nahezu verzögerungsfrei arbeiten. Eine geregelte Konstanthaltung der Brenngaseigenschaften reagiert hierbei potenziell zu träge, so, dass anstelle einer im geschlossenen Regelkreis arbeitenden Regelung ein gesteuertes Eingreifen in einer offenen Steuerkette zu bevorzugen ist.

### Darstellung der Erfindung

Der Erfindung liegt daher die Aufgabe zugrunde, ein Verfahren der eingangs genannten Art anzugeben, welches die Nachteile des Standes der Technik zu vermeiden vermag. Das Verfahren soll insbesondere geeignet sein, beim Betrieb einer Gasturbogruppe mit Brenngas auf Änderungen der massgeblichen Brennstoffeigenschaften zu reagieren. Diese Reaktion muss schnell genug erfolgen, um beispielsweise bei starken Variationen des C₂₊-Alkangehaltes einen Flammenrückschlag zu vermeiden.

Die Aufgabe wird unter Verwendung der Gesamtheit der Merkmale des Anspruchs 1 gelöst. Vorteilhafte und bevorzugte Ausführungsformen ergeben sich aus den abhängigen Unteransprüchen.

Kern der Erfindung ist es also, an der Brenngaszuführung zur Gasturbogruppe eine mit Vorteil verzögerungsfrei in Echtzeit arbeitende Vorrichtung zur Bestimmung von Brenngaseigenschaften anzuordnen, und mehrere so ermittelte Brenngaseigenschaften en in das Regelsystem der Gasturbogruppe einzubinden. In Abhängigkeit von der Brenngaseigenschaft werden dann gezielte Eingriffe auf Parameter der Gasturbogruppe vorgenommen, welche die Verbrennung beeinflussen.

Die massgebliche Brennstoffeigenschaft ist insbesondere der C₂₊-Alkan-Gehalt des Brennstoffes, welcher unmittelbar die Gefahr eines Flammenrückschlags beeinflusst. Daneben kann auch unmittelbar der Heizwert oder der Wobbe-Index als weitere Brenngaseigenschaft bei der Durchführung des Verfahrens Verwendung finden.

Eine wesentliche Voraussetzung für die Funktion des Verfahrens ist, dass die Messung sehr schnell erfolgt, und, dass die Messwerte möglichst in Echtzeit zu Verfügung stehen. In einer bevorzugten Ausführungsform findet daher eine Infrarotmesstechnik Anwendung, wie sie von Hoppe und Wolf in "IR Instrument For Gas Property Determination In Industrial Processes", IGRC 2001, Amsterdam, 6.11.2001 vorgestellt wurde. Dabei wird vorgeschlagen, die Infrarorabsorption durch das Brenngas in zwei verschiedenen Spektralbereichen zu bestimmen. Vorgeschlagen wird, die Absorption in einem ersten Spektralbereich um 3,5 µm zu bestimmen, welche primär auf den Gehalt an Äthan, Propan, und Butan reagiert, sowie in einem zweiten Spektralbereich um 7,9 µm, wo die Absorption im Wesentlichen auf den Methangehalt des Messgases reagiert. Die Querempfindlichkeit zwischen den Spektralbereichen ist gering, und kann im Bedarfsfalle leicht korrigiert werden. Auf diese Weise kann der C₂₊-Alkan-Gehalt kontinuierlich und schnell bestimmt werden. Prinzipiell genügt zur Ausführung des erfindungsgemässen Verfahrens auch die Messung nur im C₂₊-Alkan-sensitiven Spektralbereich um 3,5 µm. Dies genügt vollkommen für eine qualitative Ermittlung einer veränderten Brenngaszusammensetzung und für eine entsprechende Reaktion; genauer und für quantitative Angaben an sich notwendig ist die Messung in beiden Wellenlängenbereichen. In einer vorteilhaften Weiterbildung des Verfahrens wird zusätzlich die Infrarorabsorption in einem Wellenlängenbereich um 4,3 µm bestimmt, welche in erster Linie auf den CO₂-Gehalt reagiert. Eine Erweiterung auf weitere Spektralbereiche, welche auf spezifische Gaskomponenten sensitiv reagieren, ist ebenfalls möglich. In einer weiteren bevorzugten Weiterbildung wird die Wärmeleitfähigkeit als Mass für den Stickstoff-(N₂)-Gehalt gemessen. Gegebenenfalls unter Anwendung von Querempfindlichkeitskorrekturalgorithmen kann die Brenngaszusammensetzung auf diese Weise sehr genau kontinuierlich und in Echtzeit bestimmt werden. Dies erlaubt auch die Ermittlung des Heizwertes oder des Wobbe-Index.

Das erfindungsgemässe Verfahren lässt sich mit Vorteil an einer Gasturbogruppe mit sequentieller Verbrennung anwenden, wie sie aus EP 620 362 bekanntgeworden ist, und mit grossem Vorteil, wenn als zweite Brennkammer eine selbstzündende Brennkammer des aus EP 620 403 oder EP 669 500 bekannten Typs angeordnet ist. Selbstzündende Brennkammern sind aufgrund der hohen Gemischtemperatur in besonderem Masse von Flammenrückschlag gefährdet. Bei einer Gasturbogruppe dieses Typs wird erfindungsgemäss in Abhängigkeit von den Brennstoffeigenschaften die Brennstoffverteilung zwischen der ersten und der zweiten Brennkammer verändert, beispielsweise dergestalt, dass bei steigendem C₂₊-Gehalt die der zweiten Brennkammer zugeführte Brennstoffmenge vermindert und die der ersten Brennstoffmenge zugeführte Brennstoffmenge entsprechend erhöht wird.

Aus EP 1 199 516 ist bekanntgeworden, bei Vormischbrennern eine zentrale Axialluftströmung variierbar zu gestalten. Im Zusammenhang mit dem erfindungsgemässen Verfahrens ist es von Vorteil, in Abhängigkeit von den so bestimmten Brennstoffeigenschaften auf diese Axialströmung einzugreifen. So kann beispielsweise bei steigendem C₂₊-Alkan-Gehalt des Brenngases die Axialströmung intensiviert werden, um so die Gefahr des Flammenrückschlags in das Brennerinnere zu vermeiden.

Vormischbrenner der heute verwendeten Typen weisen häufig mehrere unabhängig voneinander mit Brennstoff beaufschlagbare Brennstoffzuführungen, beispielsweise zur unabhängigen Beaufschlagung mit Pilotbrennstoff, welcher in einer Diffusionsverbrennungsmode verbrannt *wird*, und *Vormischbrennstoff*, auf. Derartige Brenner zeigen zum Beispiel WO 01/96785, EP 193 838, EP 108 361, WO 00/12936, EP 945 677, oder EP 321 809. In Abhängigkeit von den gemessenen Brennstoffeigenschaften kann die Aufteilung des Brennstoffs verändert werden, um einerseits eine hinreichende Flammenstabilität zu gewährleisten und gleichzeitig den Flammenrückschlag oder eine Bauteilüberhitzung zu vermeiden und die Emissionswerte in etwa konstant zu halten.

Innerhalb eines Mehrbrennersystems, welches dem Fachmann geläufig ist, wird in einer Ausführungsform der Erfindung die Brennstoffverteilung zwischen einzelnen Brennern und/oder Brennergruppen in Abhängigkeit von den gemessenen Brennstoffeigenschaften variiert.

Ebenso kann in Abhängigkeit von den Brennstoffeigenschaften ein inertes Medium entweder in das Brenngas eingebracht werden, wobei diese Einbringung bevorzugt möglichst nahe an der Gasturbogruppe erfolgt, was geringe Reaktionszeiten gewährleistet. Weiterhin kann ein inertes Medium, insbesondere Dampf oder Wasser, in Abhängigkeit von den gemessenen Brennstoffeigenschaften in die Verbrennungszone eingebracht werden. Letztere Massnahme eignet sich insbesondere bei Gasturbogruppen, welche ohnehin mit Wasser- und/oder Dampfeinspritzung zur Emissionskontrolle ausgestattet sind, da sich diese auch zur Steuerung der Flammenposition und der Verbrennungsstabilität gut geeignet erweist. Grundsätzlich käme beispielsweise auch Stickstoff oder Kohlendioxid als inertes Medium in Frage, allerdings sind Wasser und Dampf in der Regel leichter verfügbar.

Eine weitere Möglichkeit zum Eingreifen besteht, wenn die Gasturbogruppe Mittel zur Kühlung des Arbeitsmittels vor dem Verdichter oder im Verdichter oder zwischen Verdichterstufen aufweist. Durch eine stärkere Kühlung wird die Temperatur der Verbrennungsluft abgesenkt, und damit die Zündwilligkeit vermindert. Es ist weiterhin bekannt, die Kühlung durch die Einbringung einer Flüssigkeit, beispielsweise von Wasser, vor dem Verdichter oder in den Verdichter zu realisieren, wobei beispielsweise Wassertropfen in den Verdichter eindringen und während der Verdichtung verdunsten. Dieses ist in der jüngeren Vergangenheit unter den Namen "Wet Compression", "High Fogging", oder "Overfogging" populär geworden, und wurde beispielsweise in der US 2,786,626 beschrieben, während die FR 1.563.749 die positiven Auswirkungen auf die Leistungsdaten einer Gasturbogruppe angibt. Die hierdurch hervorgerufene Auffeuchtung der Verbrennungsluft vermindert die Zündwilligkeit weiter.

In einer weiteren Ausführungsform der Erfindung wird in Abhängigkeit von den gemessenen Brennstoffeigenschaften unmittelbar in das Schutzsystem der Gasturbogruppe eingegriffen, dergestalt, dass beispielswiese bei einer Überschreitung eines bestimmten C₂₊-Alkan-Konzentration die Brennstoffzufuhr oder der Leistungssollwert der Gasturbogruppe vermindert werden. Weiterhin kann bei Überschreiten eines weiteren Grenzwertes ein Schutzeingriff derart erfolgen, dass eine unmittelbare Abschaltung der Gasturbogruppe erfolgt.

Selbstverständlich lassen sich diese unterschiedlichen Arten des Eingriffes auf die Gasturbogruppe auch ohne Weiteres unter Berücksichtigung der anlagenspezifische Gegebenheiten kombinieren. Es ist im Rahmen der Erfindung insbesondere auch möglich, für die Durchführung der Eingriffe auf den Betrieb der Gasturbogruppe die gemessenen Brenngaseigenschaften mit anderen gemessenen Werten der Gasturbogruppe wie Pulsations- und/oder Emissionsmesswerten, mit einer gemessenen Flammenposition, mit Materialtemperaturen, und dergleichen zu kombinieren.

### Kurze Beschreibung der zeichnungen

Die Erfindung wird nachfolgend anhand von Ausführungsbeispielen in Verbindung mit den Zeichnungen näher erläutert. Es zeigen:
Figur 1 eine Gasturbogruppe mit sequentieller Verbrennung zum Betrieb gemäss dem erfindungsgemässen Verfahren;
Figur 2 eine erste Brennerbauart und deren Betrieb gemäss dem erfindungsgemässen Verfahren;
Figur 3 eine zweite Brennerbauart und deren Betrieb gemäss dem erfindungsgemässen Verfahren;
Figur 4 eine dritte Brennerbauart und deren Betrieb gemäss dem erfindungsgemässen Verfahren;
Figur 5 ein Mehrbrennersystem und dessen Betrieb gemäss dem erfindungsgemässen Verfahren; und
Figur 6 ein weiteres Beispiel für eine erfindungsgemäss betriebene Gasturbogruppe.

Die Ausführungsbeispiele und die Figuren sind rein instruktiv zu verstehen und sollen nicht zur Einschränkung der in den Ansprüchen gekennzeichneten Erfindung herangezogen werden.

### Wege zur Ausführung der Erfindung

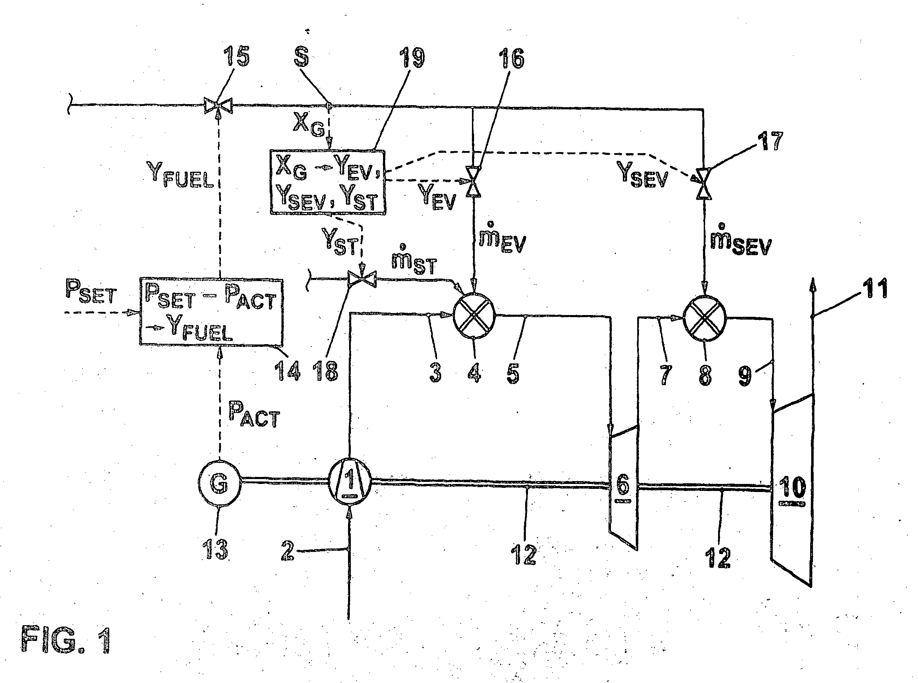

In Figur 1 ist ein erstes Beispiel für die Ausführung des erfindungsgemässen Verfahrens dargestellt. Ein Verdichter 1, eine erste Turbine 6, und eine zweite Turbine 10 sind auf einer gemeinsamen Welle 12 angeordnet. Weiterhin ist ein Generator 13 an den gleichen Wellenstrang gekoppelt. Der Verdichter 1 saugt Umgebungsluft 2 an. Diese wird verdichtet und strömt als verdichtete Brennluft 3 einer ersten Brennkammer 4 zu. Typischerweise, aber keineswegs einschränkend, kann es sich hierbei um eine Brennkammer handeln, welche mit Vormischbrennern der der oben zitierten Art ausgestattet ist. In der Brennkammer 4 wird der Verbrennungsluft 3 eine Brennstoffmenge ṁ_{EV} zugemessen und verbrannt. Dabei entstehendes heisses und gespanntes Rauchgas 5 strömt der ersten Turbine 6 zu und wird dort unter Abgabe einer Wellenleistung teilweise entspannt, typischerweise mit einem Druckverhältnis von 2. Teilentspanntes Rauchgas 7 tritt mit immer noch hoher Temperatur aus der Turbine 6 ab und strömt einer zweiten Brennkammer 8 zu. Es kann sich hierbei beispielsweise um eine Brennkammer des aus EP 669 500 bekannten Typs handeln. Dem Rauchgas 7, das einen Sauerstoffgehalt von rund 15% bis 17 % aufweist, wird eine weitere Brennstoffmenge ṁ_{SEV} zugemessen und in der Brennkammer 8 verbrannt. Das nacherhitzte Rauchgas 9 strömt einer zweiten Turbine 10 zu, und wird beim Durchströmen der zweiten Turbine 10 abermals unter Abgabe einer Wellenleistung entspannt, diesmal in etwa auf Umgebungsdruck. Es handelt sich im Grunde um eine Gasturbogruppe der aus EP 620 362 bekannten Bauart, welche Schrift in dieser Hinsicht einen integrierenden Bestandteil der Beschreibung darstellt. Das Abgas 11 weist immer noch eine Temperatur von einigen 100°C auf, und dieses Abwärmepotenzial kann auf an sich bekannte und hier nicht dargestellte Weise weiter genutzt werden. Die Wellenleistungsabgabe der Turbinen 6 und 10 dient zum Antrieb des Verdichters 1 und des Generators 13. Der Generator 13 erzeugt dabei eine elektrische Nutzleistung P_{ACT}. Ein Nutzleistungssignal wird in einem ersten Regler 14 mit einer Soll-Leistung P_{SET} verglichen. Aus der Regelabweichung P_{SET}-P_{ACT} wird eine Brennstoffmengen-Stellgrösse Y_{FUEL} gebildet, welche auf ein Brennstoffmengen-Stellorgan 15 wirkt, und auf diese Weise die gesamte Brennstoffzufuhr zu den Brennkammern 4, 8 der Gasturbogruppe steuert. Zwei Stellorgane 16 und 17 bewerkstelligen die Aufteilung der gesamten Brennstoffmenge auf die beiden Brennkammern 4 und 8.

Die Kriterien, nach denen diese Brennstoffmengenaufteilung im Allgemeinen erfolgt, sind an anderen Orten ausführlich beschrieben. Gemäss der Erfindung ist in der Gaszufuhrleitung ein Sensor S zur Ermittlung der Brenngaseigenschaften X_{G} angeordnet. Aus den Brennstoffeigenschaften X_{G} werden in einem Funktionsblock 19 Stellgrössen Y_{EV}, Y_{SEV}, und Y_{ST} gebildet. Die Stellgrösse Y_{EV} wirkt auf das Stellorgan 16, und steuert damit die Brennstoffmenge ṁ_{EV} der ersten Brennkammer 4. Die Stellgrösse Y_{SEV} wirkt auf das Stellorgan 17 und steuert damit die Brennstoffmenge ṁ_{SEV} der zweiten Brennkammer 8. Die Stellgrösse Y_{ST} wirkt auf ein Stellorgan 18, welche einen Massenstrom ṁ_{ST} inerten Mediums, beispielsweise Dampf, zur ersten Brennkammer 4 zumisst. Eine solche Dampfeinspritzung in die Brennkammer wird beispielsweise zur Emissionskontrolle als dem Fachmann geläufiger Stand der Technik eingesetzt. Erfindungsgemäss erfolgt folgender Regelungsablauf: In einem ersten Betriebszustand ist die Gasturbogruppe auf ihre Soll-Leistung eingeregelt. Die Gesamtbrennstoffmenge wird über das Stellorgan 15 eingestellt. Mittels der Stellorgane 16 und 17 erfolgt die Aufteilung des Brennstoffes auf die beiden Brennkammern 4 und 8 gemäss an anderen Orten detailliert beschriebenen Betriebskonzepten. Die Messvorrichtung S misst kontinuierlich oder quasi-kontinuierlich und nahezu ohne Zeitverzug die Brennstoffeigenschaften X_{G}, insbesondere den Gehalt an höherwertigen gesättigten Kohlenwasserstoffen mit 2 und mehr Kohlenstoffatomen, den sogenannten C₂₊-Alkanen oder gesättigten NMCH. Wenn der Gehalt an C₂₊-Alkanen im Brennstoff steigt, steigt die Zündwilligkeit des Brennstoffs und damit die Gefahr des Flammenrückschlags, insbesondere in einer Brennkammer vom selbstzündenden Typ. Daher wird bei einem gemessenen Anstieg der C₂₊-Alkan-Konzentration im Brenngas über die Stellgrössen Y_{EV} und Y_{SEV} auf die Brennstoffaufteilung auf die beiden Brennkammern 4 und 8 eingegriffen, derart, dass der Brennstoffmassenstrom ṁ_{SEV} der zweiten Brennkammer vermindert und der Brennstoffmassenstrom ṁ_{EV} der ersten Brennkammer 4 im gleichen Masse erhöht wird. Die Gefahr des Flammenrückschlags in der zweiten Brennkammer 8 wird damit ausgeräumt. Je nach Betriebszustand steigt nunmehr aber die Gefahr des Flammenrückschlags in der ersten Brennkammer 4. Daher wird über die Stellgrösse Y_{ST} das Stellorgan 18 geöffnet, und es wird eine Dampfmenge ṁ_{ST} in die erste Brennkammer 4 eingebracht, wodurch auch hier der höheren Zündwilligkeit des Brenngases Rechnung getragen wird. Wenn der C₂₊-Alkan-Gehalt des Brenngases einen Grenzwert überschreitet, wird weiterhin mit Vorteil auf nicht dargestellte, dem Fachmann aber geläufige Weise auf die Schutzsysteme der Gasturbogruppe eingegriffen, indem beispielsweise der Leistungs-Sollwert P_{SET} automatisch vermindert wird. Weiterhin kann bei Überschreiten eines weiteren Grenzwertes eine Schnellabschaltung der Gasturbogruppe erfolgen.

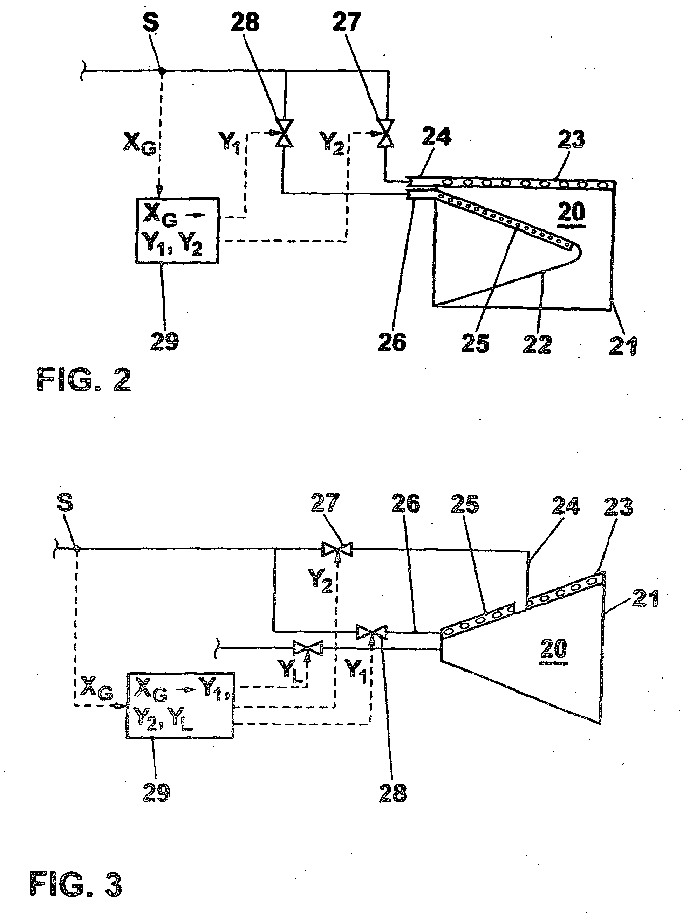

Figur 2 zeigt einen ersten Brenner für eine Gasturbogruppe einer aus WO 01/96785 bekannten Bauart in Verbindung mit einem erfindungsgemässen Betriebsverfahren. Der Brenner 20 umfasst einen zylindrischen Drallerzeuger 21 und einen konischen Innenkörper 22. Ferner weist der Brenner zwei unterschiedliche Gruppen von unabhängig voneinander mit Brennstoff beaufschlagbaren Brenngasöffnungen 23 und 25 auf, welche durch getrennte Brenngasanschlüsse 24 und 26 mit Brennstoff versorgt werden. Die Zumessung von Brennstoff zu den Brenngasöffnungen 23 und 25 erfolgt durch zwei Stellorgane 27 und 28. Deren Zuleitungen zweigen von einer gemeinsamen Brenngaszuleitung ab, in der eine Messvorrichtung S zur Bestimmung der Brenngaseigenschaften X_{G} angeordnet ist. In einem Funktionsblock 29 werden ausgehend von den Brennstoffeigenschaften X_{G} Stellgrössen Y₁ und Y₂ gebildet, welche auf die Stellorgane 27 und 28 wirken. Beim Betrieb des dargestellten Brenners in einer Brennkammer einer Gasturbogruppe werden die Stellorgane 27 und 28 nach spezifischen Kriterien angesteuert, um so eine jeweils günstige Aufteilung der Brennstoffmenge auf die Gruppen von Brenngasöffnungen 23 und 25 zu erreichen. Der Sensor S bestimmt kontinuierlich die Brenngaseigenschaften X_{G} und bei entsprechenden Veränderungen wird auf die Stellorgane 27 und 28 eingegriffen, um die Brennstoffaufteilung innerhalb des Brenners in geeigneter Weise zu verändern.

Figur 3 zeigt eine weitere aus WO 01/96185 bekannte Brennerbauart. Der Brenner 20 weist einen konischen Drallerzeuger 21 auf, wie dies aus der EP 321 809 bekanntgeworden ist. Der Brenner weist zwei Gruppen 23 und 25 von Brenngasöffnungen auf. Über die Zuleitungen 24 und 26 mit den Stellorganen 27 und 28 können die Gruppen unabhängig voneinander mit Brenngas beaufschlagt werden. Stromauf der Stellorgane 27 und 28 zweigen die Anschlüsse von einer gemeinsamen Gasversorgungsleitung ab. Der dargestellte Brenner weist weiterhin eine variierbare zentrale Axialluftzuführung auf, wie sie aus EP 1 199 516 bekanntgeworden ist. Im der Gasversorgungsleitung ist eine Messvorrichtung S angeordnet, der die Brenngaseigenschaften ermittelt und diese an die Einheit 29 liefert. Beim Betrieb innerhalb der Brennkammer einer Gasturbogruppe werden die Brennstoffmengenaufteilung auf die Gruppen 23 und 25 sowie der Axialluftstrom in geeigneter Weise vorgewählt. Bei Veränderungen der Brenngaseigenschaften wird in Abhängigkeit von den gemessenen Brenngaseigenschaften X_{G} über die Stellgrössen Y₁ und Y₂ auf die Brennstoffverteilung und über die Stellgrösse Y_{L} auf die Axialluftströmung Einfluss genommen. Insbesondere kann bei steigendem Gehalt an C₂₊-Alkanen über die Stellgrösse Y_{L} die zentrale Axialluftströmung verstärkt werden. Damit erfolgt die Flammenstabilisierung weiter stromab der Brennermündung, und die Gefahr des Flammenrückschlags wird vermieden.

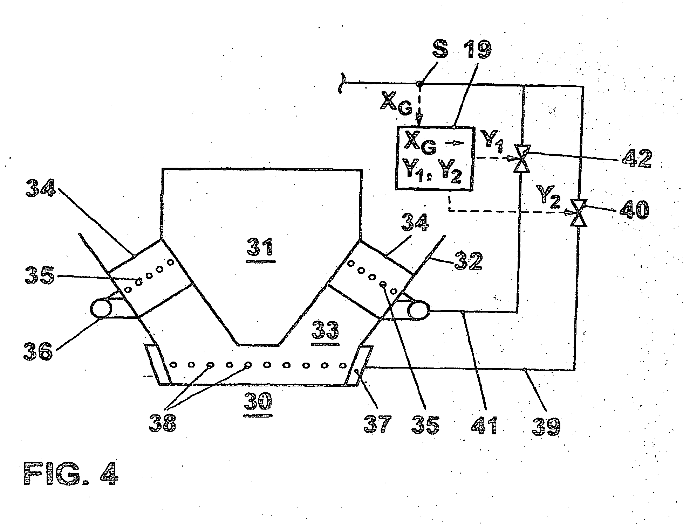

Figur 4 zeigt einen aus WO 00/12936 bekannten Brenner. Dieser weist zwei Gruppen von Brenngasöffnungen 35 und 38 auf, welche über die Ringkanäle 36 und 37, die Zuleitungen 39 und 41, sowie die Stellorgane 40 und 42 unabhängig voneinander mit Brennstoff beaufschlagbar sind. Es erfolgt, analog zu den vorstehenden Beispielen, eine Ermittlung der Brenngaseigenschaften X_{G}, und in Abhängigkeit davon eine Beeinflussung der Brennstoffaufteilung auf die Gruppen von Brenngasöffnungen 35 und 38 in Analogie zum oben beschriebenen Vorgehen.

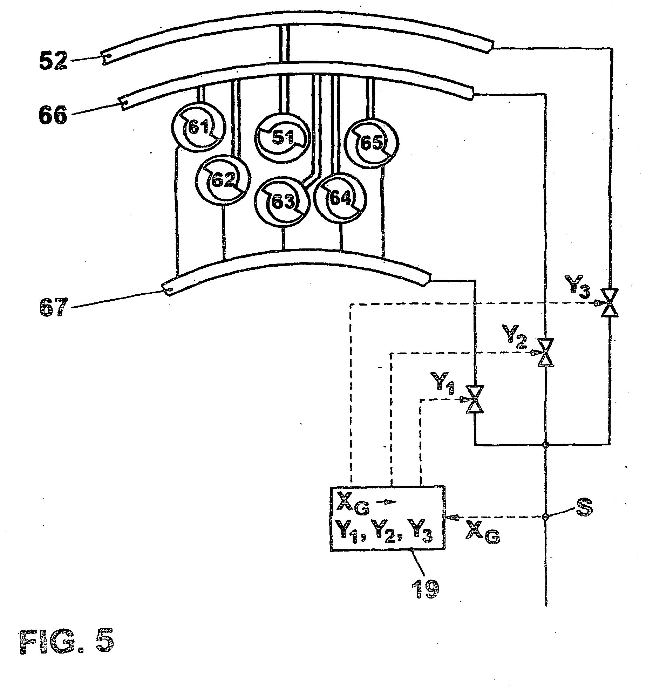

In Figur 5 ist ein Ausschnitt eines Mehrbrennersystems einer Brennkammer einer Gasturbogruppe dargestellt. Der Brenner 51 ist an eine Ringleitung 52 angeschlossen. Über diese Ringleitung werden Brenngasöffnungen für den Vormischbetrieb des Brenners angespiessen. Die Brenner 61, 62, 63, 64, 65 sind an Brennstoffleitungen 66 und 67 angeschlossen. Durch die Ringleitung 66 werden erste Gruppen von Brenngasöffnungen der Brenner 61, 62, 63, 64, 65 mit Brenngas versorgt, welche beispielsweise zur Eindüsung von Gas zur Vormischverbrennung ausgebildet sind. Durch die Ringleitung 67 werden weitere Brenngasöffnungen der Brenner 61, 62, 63, 64, 65 mit Brenngas versorgt, welche beispielsweise zur Einbringung eines in einer Diffusionsverbrennungsmode zu verbrennenden Brenngases ausgebildet sind. Die Ringleitungen ihrerseits sind an einer gemeinsamen Gasversorgung angeschlossen. Über Stellorgane können die Brenngasmassenströme, die den einzelnen Ringleitungen und den ihnen zugeordneten Brennern oder Brennergruppen oder Brenngasöffnungen zuströmen, unabhängig voneinander eingestellt werden. Ein solches Betriebskonzept ist beispielsweise von der Gasturbine GT13E2 der Anmelderin bekannt, wobei die Aufteilung der Brennstoffmassenströme auf die Ringleitungen im Wesentlichen leistungsabhängig erfolgt. Gemäss der Erfindung ist ein Sensor S zur Bestimmung der Brenngaseigenschaften X_{G} in der gemeinsamen Brenngasleitung angeordnet. Aus den Brenngaseigenschaften X_{G} werden Stellgrössen Y₁, Y₂, und Y₃ gebildet, welche auf die Brennstoffmengen-Stellorgane der Ringleitungen wirken. Auf diese Weise kann wiederum bei einer Veränderung der Brenngaseigenschaften auf die Verteilung des Brennstoffes innerhalb des Brennersystems eingegriffen werden.

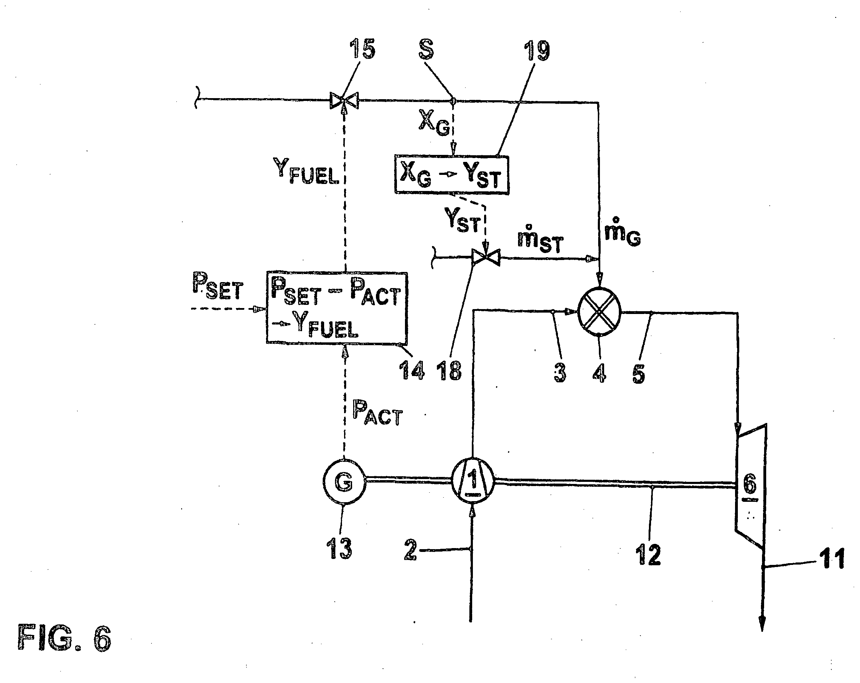

Figur 6 zeigt abschliessend eine nicht erfindungsgemässe Gasturbogruppe, deren Funktion im Lichte der vorstehenden Ausführungen nicht weiter erläutert werden muss. Ein Brennstoffmengenregler 14 regelt über die Brennstoffmengen-Stellgrösse Y_{FUEL} und das Brennstoffmengen-Stellorgan 15 den BrennstoffMassenstrom zur Brennkammer 4 so ein, dass die Regelabweichung der Leistung, P_{SET}-P_{ACT} gerade ausgeregelt ist und also verschwindet. In der Brenngasleitung ist eine Messvorrichtung S zur Bestimmung der Brenngaseigenschaften X_{G} angeordnet. In Abhängigkeit von den ermittelten Werten wird eine Stellgrösse Y_{ST} gebildet, welche auf die Stellung des Stellorgans 18 wirkt. Diese bestimmt wiederum einen Inertmedienmassenstrom ṁ_{ST} welche dem Brenngas stromauf der Einbringung in die Brennkammer 4 zugemischt wird. Wenn nunmehr beispielsweise der NMCH-Gehalt des Brenngases ansteigt, und/oder dessen Heizwert, wird an dieser Stelle Dampf oder ein anderes inertes Medium zugemischt, um die Zündwilligkeit oder den Heizwert des Gases wieder zu vermindern. Zu betonen ist, dass diese Einbringung von Inertmedien gesteuert abläuft und nicht auf die Messstelle S zurückwirkt. Dies unterscheidet das erfindungsgemässe Verfahren grundlegend beispielsweise von der Regelung einer Gasmischstation im geschlossenen Regelkreis. Während letztere vergleichsweise träge arbeitet, vermag das erfindungsgemässe Vorgehen nahezu ohne Zeitverzug zu reagieren, da ja die Messstelle stromauf der Medienzumischung angeordnet ist. Eine Veränderung der Brenngaseigenschaften wird daher eine Zeitspanne vor deren Wirksamwerden in der Brennkammer registriert. Da die Stelle der Inertmedienzumischung aber wesentlich näher an der Brennkammer angeordnet ist, ist die Zeit bis zum Wirksamwerden des Eingriffes gering. Damit ist das Verfahren ungleich besser geeignet um Gegenmassnahmen gegen drohende Maschinenschäden aufgrund der veränderten Brenngaszusammensetzung einzuleiten.

Die angeführten Ausführungsbeispiele können selbstverständlich nur einen kleinen Teil der in den Ansprüchen gekennzeichneten Erfindung abdecken. Insbesondere können die dargestellten Verfahrensvarianten in einer Vielzahl sinnvoller Kombinationen Anwendung finden. Möglich wäre auch eine Kombination der gemessenen Brennstoffeigenschaften mit Pulsations- und/oder Emissionsmesswerten, gemessenen Flammenpositionen, Materialtemperaturen und dergleichen zu Bildung der genannten Stellgrössen. Dem Fachmann eröffnen sich im Lichte der oben gemachten Ausführungen ohne erfinderisches Zutun eine Vielzahl möglicher und jeweils maschinenspezifisch auszuwählender Verfahrensvarianten.

### Bezugszeichenliste

- 1: Verdichter
- 2: Ansaugluft
- 3: verdichtete Luft, Brennluft
- 4: Brennkammer
- 5: gespanntes Rauchgas
- 6: Turbine
- 7: teilentspanntes Rauchgas
- 8: Brennkammer
- 9: nacherhitztes Rauchgas
- 10: Turbine
- 11: Abgas
- 12: Welle
- 13: Generator
- 14: Regler
- 15: Stellorgan
- 16: Stellorgan
- 17: Stellorgan
- 18: Stellorgan
- 19: Funktionsblock
- 20: Brenner
- 21: Drallerzeuger
- 22: Innenkörper
- 23: Gruppe von Brenngasöffnungen
- 24: Brenngaszuführung
- 25: Gruppe von Brenngasöffnungen
- 26: Brenngaszuführung
- 27: Stellorgan
- 28: Stellorgan
- 29: Funktionsblock
- 30: Brenner
- 31: Brenner-Innenkörper
- 32: Brenner-Aussengehäuse
- 33: Brenner-Ringraum
- 34: Brennluft-Leitschaufel, Drallerzeuger
- 35: Brenngasöffnungen
- 36: Brenngas-Ringleitung
- 37: Brenngas-Ringraum

- 38: Brenngasöffnungen
- 39: Brenngasleitung
- 40: Stellorgan
- 41: Brenngasleitung
- 42: Stellorgan
- 51: Brenner
- 52: Brenngas-Ringleitung
- 61: Brenner
- 62: Brenner
- 63: Brenner
- 64: Brenner
- 65: Brenner
- 66: Brenngas-Ringleitung
- 67: Brenngas-Ringleitung
- ṁ_{EV}: Brenngas-Massenstrom
- ṁ_{SEV}: Brenngas-Massenstrom
- ṁ_{G}: Brenngas-Massenstrom
- ṁ_{ST}: Inertmedien-Massenstrom, Dampfmassenstrom
- P_{ACT}: Nutzleistung
- P_{SET}: Soll-Leistung
- S: Messvorrichtung für Brenngaseigenschaften
- X_{G}: Messsignal für Brenngaseigenschaften
- Y₁: Stellgrösse
- Y₂: Stellgrösse
- Y₃: Stellgrösse
- Y_{FUEL}: Brennstoffmengen-Stellgrösse
- Y_{EV}: Stellgrösse
- Y_{SEV}: Stellgrösse
- Y_{ST}: Stellgrösse

## Patentansprüche

1. Verfahren zum Betrieb einer Gasturbogruppe, welcher Gasturbogruppe ein Brenngas zugeführt wird, wobei in wenigstens einer Brenngaszuführung eine Vorrichtung (S) zur Bestimmung von Brenngaseigenschaften angeordnet ist, welches Verfahren umfasst, mittels der Vorrichtung wenigstens eine massgebliche Brenngaseigenschaft (X_{G}) zu bestimmen, die massgebliche Brennstoffeigenschaft dem Regelsystem der Gasturbogruppe zuzuführen und in Abhängigkeit von der wenigstens einen massgeblichen Brennstoffeigenschaft auf die Aufteilung eines Brenngasmassenstroms auf unterschiedliche Brenngasaustrittsöffnungen eingewirkt wird, **dadurch gekennzeichnet, dass** in Abhängigkeit von den ermittelten Brenngaseigenschaften die Brennstoffverteilung zwischen einer ersten (4) Brennkammer und einer zweiten Brennkammer (8) einer Gasturbogruppe mit sequentieller Verbrennung variiert wird oder in Abhängigkeit von den ermittelten Brenngaseigenschaften die Brennstoffverteilung innerhalb eines Brenners mit mehreren Brennstoffzuführungen (23, 25, 35, 38) variiert wird oder in Abhängigkeit von den ermittelten Brenngaseigenschaften die Brennstoffverteilung innerhalb eines Mehrbrennersystems (51; 61, 62, 63, 64, 65) variiert wird.

2. Verfahren nach Anspruch 1, **dadurch gekennzeichnet, dass** der Gehalt an C₂₊-Alkanen als massgebliche Brenngaseigenschaft verwendet wird.

3. Verfahren nach Anspruch 2, **dadurch gekennzeichnet, dass** der Heizwert oder der Wobbe-Index als weitere Brenngaseigenschaft verwendet wird.

4. Verfahren nach einem der vorstehenden Ansprüche, **dadurch gekennzeichnet, dass** zur Bestimmung der Brenngaseigenschaften die Infrarotabsorption in wenigstens zwei Spektralbereichen erfasst werden, wobei die Spektralbereiche so gewählt werden, dass die Absorption in einem ersten Spektralbereich durch C₂₊-Alkane verursacht wird, und die Absorption im zweiten Spektralbereich durch Methan verursacht wird.

5. Verfahren nach Anspruch 5, **dadurch gekennzeichnet, dass** die Infrarotabsorption in wenigstens einem dritten Spektralbereich erfasst wird, wobei der dritte Spektralbereich so gewählt wird, dass die Absorption durch Kohlendioxid verursacht wird.

6. Verfahren nach einem der Ansprüche 4 oder 5, **dadurch gekennzeichnet, dass** die Wärmeleitfähigkeit des Brenngases bestimmt wird.

7. Verfahren nach einem der vorstehenden Ansprüche, **dadurch gekennzeichnet, dass** in Abhängigkeit von den ermittelten Brenngaseigenschaften die Brennstoffverteilung zwischen der ersten (4) Brennkammer und der zweiten Brennkammer (8) einer Gasturbogruppe mit sequentieller Verbrennung variiert wird, und mit steigendem C₂₊-Alkan-Gehalt der der zweiten Brennkammer zugeführte Brennstoffmassenstrom (ṁ_{SEV}) vermindert und der der ersten Brennkammer (4) zugeführte Brennstoffmassenstrom (ṁ_{EV}) entsprechend erhöht wird.

8. Verfahren nach einem der vorstehenden Ansprüche, **dadurch gekennzeichnet, dass** in Abhängigkeit von den ermittelten Brennstoffeigenschaften eine zentrale Axial-Luftströmung eines Vormischbrenners variiert wird.

9. Verfahren nach einem der vorstehenden Ansprüche, **dadurch gekennzeichnet, dass** in Abhängigkeit von den ermittelten Brennstoffeigenschaften auf die Zumischung eines inerten Mediums (ṁ_{ST}), insbesondere von Dampf, in das Brenngas eingegriffen wird.

10. Verfahren nach einem der vorstehenden Ansprüche, **dadurch gekennzeichnet, dass** in Abhängigkeit von den ermittelten Brennstoffeigenschaften auf die Zumischung eines inerten Mediums (ṁ_{ST}), insbesondere Dampf oder Wasser, zur Verbrennungsluft oder in die Verbrennungszone eingegriffen wird.

11. Verfahren nach einem der vorstehenden Ansprüche, **dadurch gekennzeichnet, dass** in Abhängigkeit von den ermittelten Brennstoffeigenschaften auf die Kühlung der Verbrennungsluft vor und/oder während der Verdichtung eingewirkt wird.

12. Verfahren nach einem der vorstehenden Ansprüche, **dadurch gekennzeichnet, dass** beim Überschreiten bestimmter gemessener Grenzwerte, insbesondere der C₂₊-Alkan-Konzentration, die Leistung der Gasturbogruppe vermindert wird.

13. Verfahren nach einem der vorstehenden Ansprüche, **dadurch gekennzeichnet, dass** beim Überschreiten bestimmter gemessener Grenzwerte, insbesondere der C₂₊-Alkan-Konzentration, eine Abschaltung der Gasturbogruppe durchgeführt wird.

## Claims

1. Method for operating a gas turbine group, which gas turbine group is supplied with a fuel gas, wherein a device (S) for determining fuel gas properties is arranged in at least one fuel gas feed, which method comprises:
by means of the device, determining at least one significant fuel gas property (X_{G}), supplying the significant fuel property to the control system of the gas turbine group, and
depending on the at least one significant fuel property, acting on the division of a fuel gas mass flow to different fuel gas outlet openings,
**characterized in that**
depending on the determined fuel gas properties, the fuel distribution between a first combustion chamber (4) and a second combustion chamber (8) of a gas turbine group with sequential combustion is varied, or
depending on the determined fuel gas properties, the fuel distribution within a burner with several fuel feeds (23, 25, 35, 38) is varied, or
depending on the determined fuel gas properties, the fuel distribution within a multiburner system (51; 61, 62, 63, 64, 65) is varied.

2. Method according to claim 1, **characterized in that** the C₂₊ alkane content is used as the significant fuel gas property.

3. Method according to claim 2, **characterized in that** the calorific value or the Wobbe index is used as a further fuel gas property.

4. Method according to any of the preceding claims, **characterized in that** to determine the fuel gas properties, the infrared absorption in at least two spectral regions is detected, wherein the spectral regions are selected such that the absorption in a first spectral region is caused by C₂₊ alkanes and the absorption in the second spectral region is caused by methane.

5. Method according to claim 4, **characterized in that** the infrared absorption in at least a third spectral region is detected, wherein the third spectral region is selected such that the absorption is caused by carbon dioxide.

6. Method according to one of claims 4 or 5, **characterized in that** the thermal conductivity of the fuel gas is determined.

7. Method according to any of the preceding claims, **characterized in that** depending on the determined fuel gas properties, the fuel distribution between the first combustion chamber (4) and the second combustion chamber (8) of a gas turbine group with sequential combustion is varied, and as the C₂₊ alkane content rises, the fuel mass flow (m_{SEV}) supplied to the second combustion chamber is reduced and the fuel mass flow (m_{EV}) supplied to the first combustion chamber (4) is increased accordingly.

8. Method according to any of the preceding claims, **characterized in that** depending on the determined fuel properties, a central axial air flow in a premix burner is varied.

9. Method according to any of the preceding claims, **characterized in that** depending on the determined fuel properties, action is taken on the admixing of an inert medium (m_{ST}), in particular steam, into the fuel gas.

10. Method according to any of the preceding claims, **characterized in that** depending on the determined fuel properties, action is taken on the admixing of an inert medium (m_{ST}), in particular steam or water, into the combustion air or into the combustion zone.

11. Method according to any of the preceding claims, **characterized in that** depending on the determined fuel properties, action is taken on the cooling of the combustion air before and/or during the compression.

12. Method according to any of the preceding claims, **characterized in that** the power of the gas turbine group is reduced if certain measured limit values, in particular for the C₂₊ alkane concentration, are exceeded.

13. Method according to any of the preceding claims, **characterized in that** the gas turbine group is shut down if certain measured limit values, in particular for the C₂₊ alkane concentration, are exceeded.

## Revendications

1. Procédé d'exploitation d'un turbo-groupe à gaz, un gaz combustible étant introduit dans ce turbo-groupe à gaz, moyennant quoi, dans au moins une alimentation en gaz combustible, est disposé un dispositif (S) pour la détermination des propriétés du gaz combustible, ce procédé comprenant la détermination, au moyen du dispositif, d'au moins une propriété de référence du gaz combustible (X_{G}), l'introduction de la propriété de référence du gaz combustible dans le système de régulation du turbo-groupe à gaz et, en fonction de l'au moins une propriété de référence du gaz combustible, on agit sur la répartition d'un flux massique de carburant sur différentes ouvertures de sortie de carburant, **caractérisé en ce que**, en fonction des propriétés déterminées du gaz combustible, la répartition du carburant entre une première chambre de combustion (4) et une deuxième chambre de combustion (8) d'un turbo-groupe à gaz avec une combustion séquentielle est modifiée, ou, en fonction des propriétés déterminées du gaz combustible, la répartition du carburant à l'intérieur d'un brûleur avec plusieurs alimentations en carburant (23, 25, 35, 38) est modifiée ou, en fonction des propriétés déterminée du gaz combustible, la répartition du carburant à l'intérieur d'un système à plusieurs brûleurs (51 ; 61, 62, 63, 64, 65) est modifiée.

2. Procédé selon la revendication 1, **caractérisé en ce que** la teneur en alcanes C₂₊ est utilisée en tant que propriété de référence du gaz combustible.

3. Procédé selon la revendication 2, **caractérisé en ce que** la valeur calorifique ou l'indice de Wobbe est utilisé comme propriété supplémentaire du gaz combustible.

4. Procédé selon l'une des revendications précédentes, **caractérisé en ce que**, pour la détermination des propriétés du gaz combustible, l'absorption des infrarouges dans au moins deux domaines spectraux est mesurée, les domaines spectraux étant choisis de façon à ce que l'absorption dans un premier domaine spectral soit provoquée par des alcanes C₂₊ et l'absorption dans le deuxième domaine spectral soit provoquée par du méthane.

5. Procédé selon la revendication 5, **caractérisé en ce que** l'absorption des infrarouges dans au moins un troisième domaine spectral est mesurée, le troisième domaine spectral étant choisi de façon à ce que l'absorption soit provoquée par du dioxyde de carbone.

6. Procédé selon la revendication 4 ou 5, **caractérisé en ce que** la conductivité thermique du gaz combustible soit déterminée.

7. Procédé selon l'une des revendications précédentes, **caractérisé en ce que**, en fonction des propriétés déterminées du gaz combustible, la répartition du carburant entre la première chambre de combustion (4) et la deuxième chambre de combustion (8) d'un turbo-groupe à gaz avec combustion séquentielle est modifiée et, lorsque la teneur en alcanes C₂₊ augmente, le flux massique de carburant (&_{SEV}) introduit dans la deuxième chambre de combustion est réduit et le flux massique de carburant (&_{EV}) introduit dans la première chambre de combustion (4) est augmenté en conséquence.

8. Procédé selon l'une des revendications précédentes, **caractérisé en ce que**, en fonction des propriétés déterminées du carburant, un écoulement d'air axial central d'un brûleur de pré-mélange est modifié.

9. Procédé selon l'une des revendications précédentes, **caractérisé en ce que**, en fonction des propriétés déterminées du carburant, il est possible d'agir sur l'ajout d'un milieu interne (&_{ST}), plus particulièrement de vapeur, dans le gaz combustible.

10. Procédé selon l'une des revendications précédentes, **caractérisé en ce que**, en fonction des propriétés déterminées du carburant, il est possible d'agir sur l'ajout d'un milieu interne (&_{ST}), plus particulièrement de vapeur ou d'eau, à l'air de combustion ou dans la zone de combustion.

11. Procédé selon l'une des revendications précédentes, **caractérisé en ce que**, en fonction des propriétés déterminées du carburant, il est possible d'agir sur le refroidissement de l'air de combustion avant et/ou pendant la compression.

12. Procédé selon l'une des revendications précédentes, **caractérisé en ce que**, lors du dépassement de certaines valeurs limites mesurées, plus particulièrement la concentration en alcanes C₂₊, la puissance du turbo-groupe à gaz est réduite.

13. Procédé selon l'une des revendications précédentes, **caractérisé en ce que**, lors du dépassement de certaines valeurs limites mesurées, plus particulièrement la concentration en alcanes C₂₊, un arrêt du turbo-groupe à gaz est effectué.
